# EUROPEAN PATENT SPECIFICATION

(11) **EP 2 255 803 B1**
(45) Date of publication and mention of the grant of the patent: **17.09.2014**
(21) Application number: 09717937.8
(22) Date of filing: 04.03.2009
(51) Int. Cl.: C07D 487/04, A61K 9/16, A61K 9/24, A61K 9/48, A61P 29/00, A61K 9/50, A61K 31/405, A61K 31/407, A61K 31/51, A61K 31/714, A61K 31/4415

(54) **ORAL GALENIC FORMULATION INCLUDING KETOROLAC AND B-COMPLEX VITAMINS, IN WHICH VITAMIN B6 IS IN AN OUTER LAYER SEPARATED FROM THE REST OF THE ACTIVE PRINCIPLES**
ORALE GALENISCHE FORMULIERUNG MIT KETOROLAC UND B-KOMPLEX-VITAMINEN MIT VITAMIN-B6 IN EINER ÄUSSEREN SCHICHT, GETRENNT VOM REST DER WIRKSTOFFE
FORMULATION GALÉNIQUE ORALE CONTENANT DU KÉTOROLAC ET DES VITAMINES DU COMPLEXE B, DANS LAQUELLE LA VITAMINE B6 SE TROUVE DANS UNE COUCHE EXTERNE SÉPARÉE DES AUTRES PRINCIPES ACTIFS

(30) Priority: 07.03.2008 MX 2008003230
(43) Date of publication of application: 01.12.2010
(73) Proprietor: Laboratorios Senosiain, S.a. De C.V., Distrito Federal 11560 (MX)
(72) Inventor: SENOSIAIN AGUILAR, Juan Aurelio, Distrito Federal 11320 (MX); GARCÍA SALGADO LÓPEZ, E. Raúl, Distrito Federal 11320 (MX); ARZOLA PANIAGUA, M. Angélica, Distrito Federal 11320 (MX); BARRANCO HERNÁNDEZ, Gustavo, Distrito Federal 11320 (MX)
(74) Representative: Carpintero Lopez, Francisco
(86) International application number: PCT/IB2009/000413
(87) International publication number: WO 2009/109836

(56) References cited:
- WO-A1-99/53928
- WO-A1-2007/116287
- FR-M- 4 492
- US-A- 4 564 614
- US-A1- 2005 232 869
- GOMES A V C ET AL: "Therapeutic properties of the B-complex vitamins (B1, B 6, B12) and their pharmacologic associations", REVISTA BRASILEIRA DE MEDICINA 200603 BR, vol. 63, no. 3, March 2006 (2006-03), pages 111-117, XP009148560, ISSN: 0034-7264
- MEDINA-SANTILLAN R. ET AL: 'B Vitamins increase the analgesic effect of Ketorolac in the formalin test in the rat' PROCEEDINGS OF THE WESTERN PHARMACOLOGY SOCIETY vol. 47, 2004, pages 95 - 99, XP009120320
- REYES G. ET AL: 'Effect of Dexamethasone Plus Vitamin B Complex in the PIFIR Model' PROCEEDINGS OF THE WESTERN PHARMACOLOGY SOCIETY vol. 43, 2000, pages 51 - 53, XP008130657

## Description

### FIELD OF THE INVENTION

The present invention refers to a solid oral pharmaceutical composition comprising the combination of ketorolac and B-complex comprising, inter alia thiamin, pyridoxine and cyanocobalamin (vitamins B1, B6 and B12, respectively) and/or the pharmaceutically acceptable salts thereof, as well as pharmaceutically acceptable vehicles and/or excipients; the manufacturing process of said composition and the use of said composition having a synergistic therapeutic activity, indicated for the treatment of moderate to severe pain or neuralgias in different locations.

### BACKGROUND OF THE INVENTION

The treatment of inflammatory-originated pain requires the use of different strategies, such as the use of non-steroidal anti-inflammatory agents (NSAIDs). However, depending on the pain extent, the use of NSAIDs alone will not provide a satisfactory pain control, and, furthermore, major adverse events could appear with the chronic use of said agents, namely gastric damage, gastric ulcers (Gambero et al., 2005).

For the present invention, a stable, effective, oral formulation was developed, having less adverse side effects, wherein an analgesic (ketorolac) known for its therapeutic efficacy and B-complex comprising thiamine (vitamin B1), pyridoxine (vitamin B6) and/or cyanocobalamin (vitamin B12), coexists. Due to physicochemical interactions occurring among active drugs of the present invention, it is difficult to create a pharmaceutical compound by simple association of active drugs.

Ketorolac tromethamine, (+-)-5(benzoyl)-2,3-dihydro-1H-pyrrolizine-1-carboxylic acid tris (hydroxymethyl)methylamine is a highly water-soluble, non-steroidal, anti-inflammatory analgesic (NSAID), having a pH from neutral to slightly basic of 9.0, and melting point from 160 to 161°C.

Ketorolac acts on the cyclooxigenase II enzyme, which mediates in the painful inflammatory process, having a plasmatic mean life from 4 to 6 hours. More than 99% is bound to proteins and about 90% is excreted without any change in urine, the remaining is excreted in the feces.

Clinical studies have demonstrated that Ketorolac is a powerful and effective analgesic (similar to an opiate type analgesic), useful in the treatment of moderate to severe pain.

In addition to its analgesic effect, it is a moderate antipyretic and, compared to opioid analgesics, it does not produce adverse effects such as: nausea, vomit, sedation or tolerance. Ketorolac is an analgesic with peripheral action that does not interfere with opiate binding and does not exacerbate sedation or respiratory depression associated with the use thereof.

Usual oral ketorolac doses are from 10 mg to 30 mg, three to four times a day, said dose may increase depending on the pharmaceutical form, severity and kind of pain.

B-complex vitamins are used in the present composition, these can be hydrosoluble and liposoluble, having a pH between 3.0 and 5.0; they are highly photosensitive and, in general, sensitive to the surrounding environment. Vitamins B1, B6, and/or B12, which are hydrosoluble vitamins, are used in the present invention, with the use thereof not limiting the use of other vitamins within the B-complex.

Thiamine (vitamin B1) is part of a coenzyme, which participates in metabolic reactions of lipids and carbohydrates; it normalizes nervous, cardiovascular and digestive system functions.

Pyridoxine (vitamin B6) promotes metabolism of lipids and proteins into amino acids, and stimulates the fagocitary activity of white blood cells.

Cyanocobalamin (vitamin B12) helps in nucleic acid formation, contributes to the normal functioning of red blood cells and helps in maintaining a well functioning state of nervous cells.

B-complex (vitamins B1, B6 and/or B12) has multiple therapeutic applications such as: vitamin deficiencies, in a pre- or post-operatory state, neuritis, polyneuritis, chronic diarrhea, polyneuropathies and Wernicke encephalopathy, among other theurapeutic activities.

Due to ketorolac and B-complex vitamins properties, when combined in a pharmaceutical oral composition, incompatibility problems arise because chemical reactions that promote degradation of the active agents ketorolac and B-complex vitamins take place, causing non-conformity of the formulation regarding stability requirements and, consequently, of the desired therapeutic effect.

It was confirmed experimentally, in the present invention, that the combination of ketorolac with B-complex vitamins, leads to the modification of ketorolac physical and chemical properties. In particular, when combined with pyridoxine (vitamin B6), a mayor formulation instability occurs, with said vitamin being mostly responsible for ketorolac degradation in the pharmaceutical combination.

Coexistente of ketorolac and B-complex vitamins in the same dose unit by simple association is not possible, since degradation can take place due to particular physicochemical properties of the active ingredients.

Currently, a known therapy used in pain treatment in the immediate post-operatory state is the use of analgesics in combination with opioids, as described in the patent application No. PA/a/2002/010828. The document refers to a pharmaceutical composition in capsule form, comprising the association of a non-steroid anti-inflammatory analgesic (NSAID), ketorolac, with an opiate, tramadol, used in pain treatment.

The disadvantage of the above composition relates to adverse side effects of the opiate tramadol, wherein the restriction of opioids usage is associated with side effects, being primarily the following: itch, nausea, vomit, urinary retention, constipation, paralytic ileum, potential respiratory depression, sedation, tolerance, anxiety, abstinence syndrome and/or respiratory depression.

Combinations of analgesics with pyridoxine (vitamin B6) exist in the state of the art, as described in the patent document WO/1999/053928. Said document discloses a method for alleviating inflammatory conditions in patients suffering from hypertension and being treated with an NSAID. The related composition comprises administering NSAID and vitamin B6, jointly or separately; specifically, combinations of diclofenac, indomethacin and acetyl salicilate with vitamin B6 are recited. It is worth mentioning that, although ketorolac is cited in said document in a general form, there is no experimental evidence in said document WO/1999/053929 about the combination of ketorolac and vitamin B6.

On the other hand, patent US 4,564,614 refers to an anti-inflammatory pharmaceutical composition useful in the treatment of gastric ailments or gastric ulcers. Said composition envisions the direct binding of piroxicam and pyridoxine (vitamin B6) in an oral tablet formulation. Unlike the present invention, therapeutic indication is different, as well as the physicochemical properties of the NSAID used. Therefore, said background does not contemplate possible interactions between an NSAID and any B-complex vitamins.

Patent application No. PA/a/2006/004020 refers to ketorolac and B-complex pharmaceutical compositions having therapeutic activity in the treatment of moderate to severe pain, and to the process for obtaining said compositions. Said invention discloses an injectable formulation and its process of manufacture, as well as a pharmaceutical formulation in capsules containing coated microspheres, wherein the objective was to keep vitamin B12 protected and isolated from physicochemical interactions with the rest of the active ingredients in the formulation, the reason being that said vitamin is less stable and is present in lesser amounts in said formulation.

Unlike the patent application No. PA/a/2006/004020, the present invention refers only to ketorolac and B-complex oral compositions wherein coexistence of ketorolac tromethamine and B-complex is achieved in the same dose unit. Active substances are contained in individual and separated compartments, using a reduced amount of excipients, which improves operation time and costs; stability is reached without the use of any protection system by means of buffers or antioxidants (such as citric acid) typically employed in pharmaceutical formulations. In the present invention vitamin B6 is applied as part of the outer layer.

Currently, there are formulations of pharmaceutical combinations comprising an NSAID (diclofenac, meloxicam or ketoprofen) and B-complex (vitamin B1, B6, and B12) or, an NSAID like ketorolac in combination with the tramadol opiate; side effects of different kind and severity may appear regarding these formulations.

Diclofenac formulations indicated for a short-term pain treatment, once in the organism, show the effect of the first step of the metabolic absorption, which leads to reaching the maximum plasma concentration in a longer period of time than ketorolac, which does not show the effect of said first metabolic step and it is absorbed immediately.

The ketoprofen and B-complex oral formulation is administered from 200 mg to 300 mg per day, the half-life of ketoprofen being from about 1 to 3 hours. When administered with meals, absorption becomes slower, resulting in a delay and reduction of the concentration peak (Cmax). Administration of ketoprofen and B-complex at the usual doses as mentioned above may lead to cefalea, confusion, cutaneous rash, nausea, vomit, and gastric irritation, and may occasionally cause Stevens-Johnson syndrome.

In the case of the meloxicam and B-complex oral formulation, its main therapeutic effect is as an anti-inflammatory, having a therapeutic indication different from the therapeutic indication of the present invention.

On the other hand, when following a pain treatment regime with NSAIDs, close supervision is recommended for those patients suffering from gastrointestinal disorders or those having a history suggesting presence of gastric or intestinal ulcer; less frequently, the following might appear: Stevens-Johnson syndrome, Lyell syndrome, erythroderma (exfoliative dermatitis), alopecia, photosensitive reactions, purpura, and in some cases allergic reactions.

In the case of pain treatment with an NSAID combined with an opiate analgesic such as tramadol, its use is more delicate, since its extended use can cause gastric injury, somnolence, headache, nausea, tolerance, anxiety, withdrawal effect, respiratory disorders, sedation, confusion, and even resistance and tolerance may appear.

For the above reasons, it is convenient to have a pharmaceutical formulation which addresses moderate to severe pain ailment without causing adverse side effects commonly related to NSAIDs. By using a lower dose of ketorolac than what is typically used, the ketorolac and B-complex oral formulation of the present invention is intended to relieve both moderate to severe pain and neuralgia in different locations, with less risk of suffering from side effects.

Unlike diclofenac, ketoprofen, tramadol formulations and combinations thereof with B-complex, the present invention, given its synergistic effect and by the use of a dose of ketorolac which is lower than the common therapeutic dose thereof, does not cause the adverse side effects commonly associated with these compositions; further, it should be noted that treatment periods may be increased when necessary, resulting in a greater effect or keeping the analgesic therapeutic effect against moderate to severe pain and neuralgias in different sites.

In the present invention, coexistence of active ingredients in the same unit dose is obtained by successfully isolating the active ingredients from direct contact in separate compartments. For the composition of the present invention we identified that vitamin B6 in presence of ketorolac shows a higher oxidation reaction, therefore we developed a separate compartment system preventing formation of this kind of interactions.

One design of the tablet composition comprises the isolation, in a first compartment, of ketorolac, vitamin B1, vitamin B12, compressibility vehicle, diluent binder, antistatic agent, lubricant, plasticizer, and a disintegrant. A coating or isolating layer, corresponding to a second compartment, is added to the first one, said layer comprising a protective coating polymer. Then, pyridoxine and a binding polymer are added in order to form a third compartment. Finally, a final coating can optionally be added, using a polymer for final coating and finishing, said polymer confers color and gloss, as well as protection against potential photolysis and/or hydrolysis.

A preferred embodiment of the present invention uses 5 mg or less of the typical ketorolac dosage (10 mg), so it is less likely that the formulation of the present invention could cause adverse side effects compared to currently existing conventional formulations that contain an NSAID.

The present formulation provides an easier administration in persons who do not wish or cannot get a treatment via parenteral administration, such as post-operatory patients, seniors or the like, since it is a small tablet of no more than one half centimeter in diameter.

### BRIEF DESCRIPTION OF THE DRAWINGS

The attached figures show drug behavior.
Figure 1 shows the timing of the analog visual scale (AVS) after administering diclofenac-vitamins B (black circles) and ketorolac-vitamins B (white circles) every eight hours.
Figure 2 shows the timing of the first eight hours of the analog visual scale (AVS) in patients who received diclofenac-vitamins B (black circles) and ketorolac-vitamins B (white circles).
Figure 3 shows the Area Under the Curve (AUC) of a 24-hour timing of the analog visual scale. Data are expressed as average ± standard error.
Figure 4 shows the Area Under the Curve (AUC) of an 8-hour timing plot of the analog visual scale. Data are expressed as average t standard error.

### DESCRIPTION OF THE INVENTION

The challenge faced by the development of the present invention is the obtention of a stable, safe, oral pharmaceutical composition having therapeutic efficacy, in a single dose unit comprising drugs physically and chemically incompatible one to another such as ketorolac and B-complex, with associated reduced operating times and excipient use.

Ketorolac monodrug oral formulations and formulations containing B-complex exist, so it could be thought that it is possible to perform a composition by simply mixing these compositions. However, this is not possible, because a plurality of physical and chemical interactions exist, which would take place if these active ingredients were contained together.

Ketorolac, due to its physical and chemical properties, has higher solution stability in basic medium, in a pH range between 7.0 and 9.5. B-complex shows a higher stability in an acid pH range between 3.0 and 5.0. These drug properties difficult the manufacturing process of the pharmaceutical composition when trying not to affect the stability of each drug. By simple combination of ketorolac-B-complex in a solution, evident physical instability and active drug degradation occur, as well as turbidity, precipitate formation and an acid pH between 3.5 and 5.0, which is an unfavorable pH for ketorolac stability.

In the present invention, coexistence of active ingredients in the same dose unit is obtained by successfully isolating the active ingredients avoiding direct contact, by including them in separate compartments. These formulations can be present in tablets or coated cores.

A design of the composition as tablets comprises isolation of ketorolac, vitamin B1, vitamin B12, compressibility vehicle, diluent binder, antistatic agent, lubricant, plasticizer, and a disintegrant, in a first compartment. A coating or isolating layer comprising a protective coating polymer corresponding to the second compartment, is added to the first one. Then, pyridoxine and a binding polymer are added to form a third compartment. Finally, a final covering may be optionally added, comprising a polymer for final coating and finishing, said polymer confers color and gloss, as well as protection against potential photolysis and/or hydrolysis.

One of the main uses of polymeric excipients in solid pharmaceutical formulations is to provide protection to said formulations during other manufacturing processes (e.g. coatings or compression) or against external factors such as, without limitation, humidity and light. In the present invention, however, it is worth mentioning that this formulation uses a polymeric coating or layer containing the active ingredient, vitamin 86, which is an uncommon process.

One way to reduce processing timing is by the inclusion of the active ingredient along with the polymer. However, application of the coating polymer with amounts of active ingredient (vitamin B6) jointly, may cause problems during processing, such as not complying with dosage uniformity. This technical difficulty represented a major challenge to the present invention, which was solved, and the formation of an homogenous film was obtained, achieving thereby the goal of containing vitamin B6 and conferring protection to the tablet.

Therefore, during the development of the pharmaceutical composition of the present invention, several technical problems were solved in order to obtain a stable and safe composition, by shielding active agents with polymeric excipients and by protecting said formulation against light intensity and the surrounding environment in general.

### Formulations

Because oral formulation is among the most widely accepted formulations due to its comfortable administration and ease of transportation, the composition of the present invention can be provided in the form of tablets.

The formulation and manufacturing process thereof is disclosed below, said formulation comprising ketorolac and B-complex active ingredients and/or pharmaceutically acceptable salts thereof, and, in addition, pharmaceutically acceptable vehicles or excipients. Weight amounts of active ingredients, vehicles and/or excipients, can be used within the ranges recited in the description of formulation compounds.

### EXAMPLE 1. ORAL FORMULATION IN PELLET, TABLET.

### Description of formulation compounds:

**Active Ingredients,** as disclosed or pharmaceutically acceptable salts thereof:
- Ketorolac tromethamine, which can be comprised from 2 mg to 40 mg per dose unit and/or from 0.2% to 6.0% by weight of the composition.
- Thiamine hydrochloride or mononitrate (vitamin B1), which can be comprised from 15 mg to 250 mg per dose unit and/or from 2.5% to 60.0% by weight of the composition.
- Pyridoxine hydrochloride (vitamin B6), which can be comprised from 15 mg to 250 mg per dose unit and/or from 2.5% to 60.0 by weight of the composition.
- Cyanocobalamin (vitamin B12), which can be comprised from 0.1 mg to 10 mg per dose unit and/or from 0.05% to 1.5% by weight of the composition.

Pharmaceutically acceptable **excipients and/or vehicles:**
- **Compressibility vehicle:** its role is as a base for active ingredients and other components of the formulation. It can be selected from: microcrystalline cellulose, lactose, starch, a mixture thereof, or another equivalent excipient, with microcrystalline cellulose being preferred. Compressibility vehicle can be present from 90.0 mg to 120 mg per unit dose and/or from 15.0% to 60.0% by weight of the composition.
- **Diluent binder:** it is used as a carrier for the active ingredients and other components of the formulation; it can be selected from: lactose, dextrose, sucrose, mannitol, with lactose being the preferred base. The excipient can be present from 50 mg to 90 mg per dose unit and/or from 5.0% to 60.0% by weight of the composition.
- **Antistatic agent:** allows for electrostatic charge suppression and helps in the flowability of the formulation during the manufacturing process, it can be selected from silicon dioxide, talc, a mixture thereof, or another equivalent excipient, with silicon dioxide being the preferred antistatic agent. The antistatic agent can be present from 0.5 mg to 3.0 mg per unit dose and/or from 0.002% to 1.0% by weight of the composition.
- **Disintegrant:** Helps in the expansion of the pharmaceutical form when being in contact with water, allowing for the compressed pellet disintegration once it enters the organism; it can be selected from sodium croscarmellose, sodium starch glycolate, mixtures thereof, or another equivalent excipient, with sodium croscarmellose being the preferred disintegrant. The disintegrant can be present from 2.0 mg to 6.0 mg per dose unit and/or from 0.5% to 2.5% by weight of the composition.
- **Lubricant:** it is selected from magnesium stearate, magnesium phosphate, stearic acid, glyceryl stearate, polyethylene glycol, sodium stearyl fumarate, talc, a mixture thereof, or another equivalent excipient, with magnesium stearate being preferred. The lubricant can be present from 3.0 mg to 5.0 mg per unit dose and/or from 0.5% to 2.5% by weight of the composition.
- **Plasticizer:** it is incorporated as a fragility reducer as well as a modifier of both film plasticity and elasticity; it is selected from polyethylene glycol, propylene glycol, among other glycol derivatives; with propylene glycol being the preferred plasticizer. The plasticizer can be present from 1.5 mg to 3.5 mg per unit dose and/or from 0.2% to 11.5% by weight of the composition.
- **Ligand binder polymer:** helps in the adhesion and cohesion of the components of the formulation, it is selected from polyvynilpyrrolidone, hydroxypropyl cellulose, hypromellose, a mixture thereof, or another equivalent excipient, with hypromellose being the preferred ligand binder polymer. The polymer can be present in said formulation from 25.0 mg to 45.0 mg per dose unit and/or from 5.0% to 40.0% by weight of the composition.
- **Coating polymer:** provides protection to the compressed pellet, improves handling and stability strength while in storage; it is selected from methacrylate copolymer derivatives (Opadry), hydroxypropylmethyl cellulose, polyvinylpyrrolidone or another equivalent excipient; with Opadry being the preferred coating polymer. The polymer can be present from 10.0 mg to 16.0 mg per unit dose and/or from 1.5% to 7.5% by weight of the composition.
- **Coating and finishing polymer:** improves appearance and is formulation-distinctive, and besides providing good appearance, it masks taste or unpleasant odors; it can be selected from celullose derivatives and methacrylates (Opaglos), polyvynilpyrrolidone, polyvinyl alcohol, hydroxypropylmethyl cellulose, polyethylene glycol or another equivalent excipient; with either polyethylene glycol or Opaglos being the preferred coating and finishing polymers. The polymer can be present from 1.0 mg to 3.0 mg per unit dose and/or from 0.5% to 7.5% by weight of the composition.

The following table shows the general formula, without limiting the formulation; it is possible to make the composition by varying compound amounts within useful permissible range limits.

**TABLE 1. GENERAL FORMULATION OF COMPRESSED PELLETS, TABLETS**

| Ingredients | Permisible limits of use milligrams/unit dose |
|---|---|
| Ketorolac tromethamine | 2 - 40 |
| Thiamine hydrochloride | 15 - 250 |
| Pyridoxine hydrochloride | 15 - 250 |
| Cyanocobalamin | 0.1 - 10 |
| Compressibility vehicle | 90 - 300 |
| Diluent binder | 50.0 - 90.0 |
| Disintegrant | 2 - 15 |
| Antistatic agent | 0.5 - 10.0 |
| Lubricant | 2.5 - 7.5 |
| Ligand binder polymer | 25 - 65 |
| Plasticizer | 1.5 - 7.5 |
| Coating polymer | 10 - 20 |
| Coating and finishing polymer | 1.0 - 3.0 |
| Purified water* | Cbp |
| Total | aprox. 340 - 1000 mg |

| | |
|---|---|
| *It evaporates during processing | |

The following example shows the object of the invention as tablets, however without limiting the invention.

**TABLE 2. ORAL FORMULATION OF COMPRESSED PELLETS, TABLETS**

| Ingredients | Formulation 1 | Formulation 2 | Formulation 3 |
|---|---|---|---|
| | Miligrams /unit dose | | |
| Ketorolac tromethamine | 2 | 5.0 | 40 |
| Thiamine hydrochloride | 15 | 50.0 | 250 |
| Pyridoxine hydrochloride | 15 | 50.0 | 250 |
| Cyanocobalamin | 0.1 | 1.0 | 10 |
| Compressibility vehicle | 300 | 110.4 | 90 |
| Diluent binder | 90 | 73 | 50 |
| Disintegrant | 15 | 4.3 | 2 |
| Antistatic agent | 10 | 1.5 | 0.5 |
| Lubricant | 7.5 | 4.3 | 2.5 |
| Ligand binder polymer | 65 | 35.7 | 25 |
| Plasticizer | 7.5 | 2.3 | 1.5 |
| Coating polymer | 20 | 13.0 | 10 |
| Coating and finishing polymer | 3.0 | 2.1 | 1.0 |
| Purified water* | cbp | cbp | cbp |
| Total | aprox. 440 mg | aprox. 440 mg | aprox. 1000 mg |

| | | | |
|---|---|---|---|
| *It evaporates during processing | | | |

A preparation method of said composition is described below. The following example shows the object of the invention by means of the tablet formulation, however it does not limit the invention to this specific example.
1. Weighing of formulation components.
2. Mixing the compressibility vehicle: 110.4 mg microcrystalline cellulose; active ingredients: 5.0 mg ketorolac, 50.0 mg thiamine, 50.0 mg cyanocobalamin; antistatic agent: 1.5 mg silicon dioxide; diluent binder: 73.0 lactose; lubricant: 4.3 mg magnesium stearate, disintegrant: 4.3 mg sodium croscarmellose; and plasticizer: 2.3 mg propylene glycol.
3. Compressing the mixture obtained from step 2.
4. Coating the compressed mixture with 34.6 mg hypromellose.
5. Separately, dissolving the active ingredient, 50 mg pyridoxine, in water.
6. Applying the solution from step 5 to the compressed mixture from step 4.
7. Applying to the compressed mixture of step 6, a coating comprising the coating polymer, preferable Opadry, 13.0 mg.
8. Optionally, applying to the tablet obtained from step 7, a solution comprising polymer and finisher, preferably 2.1 mg polyethylene glycol or 2.1 mg Opaglos.
9. Conditioning the tablet.

The formulations in coated compressed tablet forms were evaluated in stability studies in order to demonstrate that said formulations are robust and can keep their stability.

Chart 1. Stability study for oral compositions in compressed pellets, tablets. These tablets were prepared according to the preferred form of formulation 2 of table 2.

The evaluated parameters were formulation appearance, worthiness and stability.

### Stability study results

| Parameter to be evaluated | Initial Time | | Final Time | |
|---|---|---|---|---|
| | Formulation by simple association of active agents | Formulation according to the present invention | Formulation by simple association of active agents | Formulation according to the present invention |
| Appearance | Excellent | Excellent | Non-Satisfactory | Excellent |
| Worthiness | 96.0 % | 96.7 % | 59-3 % | 93.0 % |
| Stability | 98.0 % | 99.2 % | 62.3 % | 94.0 % |

Wherein, excellent, satisfactory and non-satisfactory mean:
Excellent: Full integrity of the tablet (circular), with an even coating or layer, tablet shows homogeneous color.
Satisfactory: Full integrity of the tablet (circular), with an even coating or layer, tablet shows opaque or non-homogeneous color.
Non-satisfactory: Porous tablet, cracked coating or layer, tablet shows non-homogeneous color.

According to the results obtained, the present invention formulations prove being stable and robust against environment conditions, indicating that they are safe for administration and effective in their therapeutic activity.

On the other hand, a comparative clinical study of the present invention formulation against a commercial diclofenac plus B-complex formulation was carried out in patients suffering from knee or hip arthrosis grade III and IV, before they were submitted to replacement surgery.

The analgesic effect of ketorolac-vitamins against diclofenac-vitamins B was compared in patients with chronic pain caused by the degenerative joint disease. This study was applied to persons within 40 and 60 years old, men or women, having pain greater than or equal to 5 according to the visual analog scale (1-10), patients were not taking any drugs that could interfere with the pharmacokinetics or efficacy of active ingredients included in the formulations of the present study, they were hospitalized as candidates scheduled for surgery.

The studied formulations were: Diclofenac in combination with vitamins B1, B6 and B12 of 50 mg, 50 mg, 50 mg and 0.25 mg (Dolo-neurobion®); and ketorolac in combination with B1, B6 and B12 of 5 mg, 50 mg, 50 mg, and 1 mg.

The study was carried out according to a parallel experimental design. Two groups of 10 patients each were formed. Patients were given ketorolac-vitamins B or diclofenac-vitamins B every 8 hours per day, and pain was recorded according to the analog visual scale.

Group 1 received a formulation containing diclofenac and B-complex (commercial formulation), while Group 2 received a formulation comprising 5 mg ketorolac, 50 mg thiamine, 50 mg pyridoxine and 1 mg cianoconalamin. It should be noted that the diclofenac and B-complex formulation used in this study contains 5 mg ketorolac which is half the typical dose used in oral commercial ketorolac formulations of 10 mg per unit dose.

Timing plots of the effect were obtained and the Area Under the Curve of said effect was calculated.

The treatment designation was made randomly and a formulation was administered every 8 hours. Pain was evaluated by means of a 10 cm analog visual scale before administering said formulations and at 0.5, 1, 2, 4, 6, 8, 12, 16, and 24 hours after drug administration. At the end of the study patients were surveyed about the overall evaluation of pain relief associated with the pain at the time they were admitted in the study.

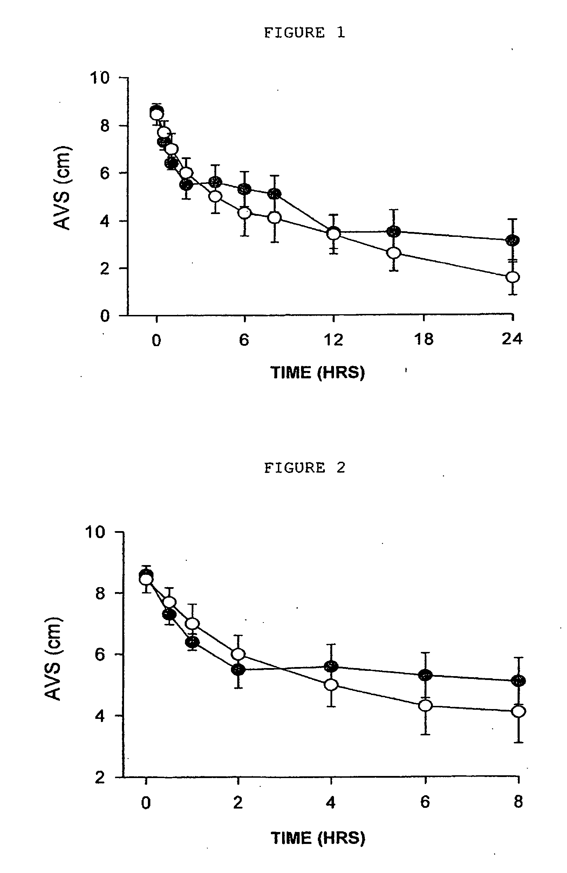

Figure 1 shows the timing plot of the analog visual scale in 10 patients who received treatment with diclofenac-vitamins B every 8 hours (black circles) and 10 patients who received ketorolac-vitamins B every 8 hours (white circles). Data are expressed as average ± standard error.

It can be seen that, in average, patients start with a pain between 8 and 9 (indicating severe pain). Under these conditions, it was observed that both treatments were able to produce a similar analgesic effect; no significant difference was encountered among these treatments.

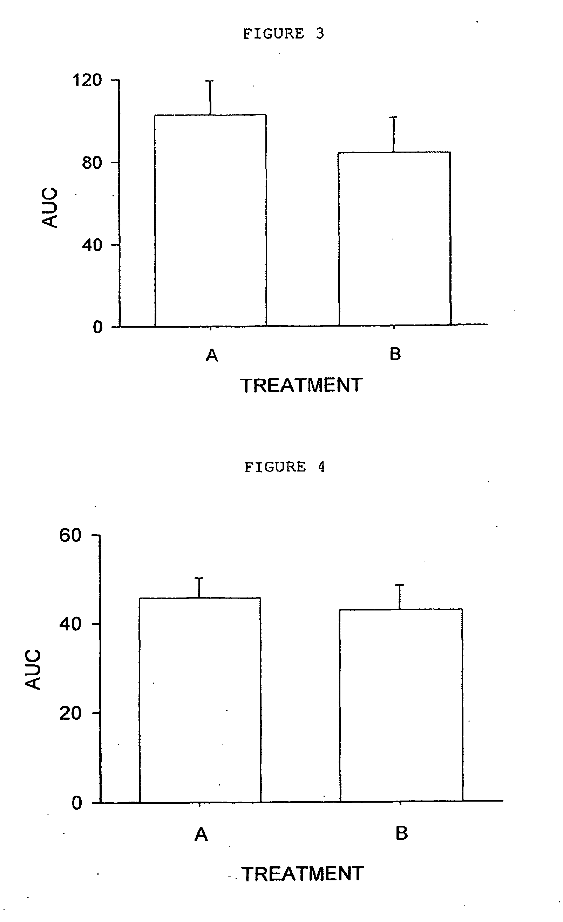

Figure 2 shows the first eight hours of the timing plot for the analog visual scale in patients having received diclofenac-vitamins B (black circles) and ketorolac-vitamin B (white circles). A similar behavior can be observed from both treatments, with a small tendency of ketorolac-vitamins B to produce a higher effect. The area under the curve (AUC) was plotted up to 24 hours (figure 3) and up to 8 hours (figure 4). In both cases, it was observed that ketorolac-vitamins B (Column A) produced a slightly higher effect in relation to diclofenac-vitamins B (Column B), most of all, at 24 hours. On the other hand, it was observed that 80% of the patients treated with the ketorolac-vitamins B combination reported full relief and 20% moderate relief; while in the case of the diclofenac-vitamins B treatment, 50% of the patients reported full relief and 50% moderate relief.

In this study, the ketorolac-vitamins B combination was evaluated regarding whether it is at least as effective as the diclofenac-vitamins B combination in a similar dosage scheme.

It is important to consider that in a typical treatment with ketorolac as a monodrug, dosage is 10 mg every 6 hours, in the present study a ketorolac-B complex combination was used wherein ketorolac is present as a 5 mg dose and administered every 8 hours. Less than half the daily ketorolac dose was administered, which will result in a reduced incidence of adverse effects.

Based on the clinical study results, it can be concluded that the combination of ketorolac and vitamins B is a good option in pain treatment and it is perfectly justified.

According to the foregoing, the present invention discloses pharmaceutical compositions indicated in the prevention and treatment of pain from moderate to severe, said compositions can be administered in a same dose unit.

Formulations presented in this invention are physicochemically stable as well as therapeutically effective, this was achieved by developing compositions that prevent direct contact between the active ingredients, which are incompatible one another, contained in separate compartments in order to avoid physicochemical interactions.

In formulating said compositions in separate compartments, wherein both active ingredients and excipients are added independently and in separately, a non-oxidative balance is obtained, preventing degrading interactions that otherwise would appear between active ingredients.

The obtained formulations do not limit the content of active ingredients to the exemplary formulations described herein, since these formulations could vary in ketorolac and B complex content. That is to say, ketorolac can be present in 5 mg, 10 mg, 15 mg, 30 mg, and up to 40 mg per unite dose. B-complex can be present from 25 mg, 50 mg, 100 mg, 150 mg, and up to 250 mg thiamine; 25 mg, 50 mg, 100 mg, 150 mg, and up to 250 mg pyridoxine; and 1 mg, 2 mg, 5 mg, and 10 mg cyanocobalamin.

A preferred formulation, without limiting the scope of the present invention, contains 5.0 mg ketorolac, 50.0 mg vitamin B1, 50 mg vitamin B6 and 1.0 mg vitamin B12.

The formulations of the present invention provide for a better control of the plasmatic level of drugs, by being evenly distributed within the gastrointestinal tract since they are readily dissolved and have immediate release, resulting in less bioavailability variations, and providing better control both in the administration regime and therapeutic monitoring.

Compared to existing products of NSAIDs alone, combinations of NSAID with B-complex and opiate with B-complex, the present invention can provide a remarkable reduction in adverse side effects; this is obtained by using a low ketorolac dosage, without compromising the therapeutic effect, and because ketorolac acts synergistically with B-complex in pain treatment.

The present invention embodied as tablets, does not limit its presentation, and can be applied to other pharmaceutical forms such as: microspheres in capsules, microcapsules, or other spherical or non-spherical particulate systems, and other solid pharmaceutical forms as compressed pellets.

In the case of microencapsulated formulations, the cited excipients used in tablets are mostly employed in addition or instead of: inert cores, antioxidant sequestrants and antioxidants, without limiting the use thereof.

**Table 3. Oral Formulation of capsules with microspheres**

| Ingredients | Range of use milligrams/unit dose |
|---|---|
| Ketorolac tromethamine | 2 - 40 |
| Thiamine hydrochloride | 15 - 250 |
| Pyridoxine hydrochloride | 15 - 250 |
| Cyanocobalamin | 0.1 - 10 |
| Inert cores | 160 - 400 |
| Coating polymer | 10 - 20 |
| Antioxidant sequestrant | 0.02 - 0.5 |
| Ligand binder polymer | 0.02 - 0.5 |
| Antioxidant | 0.001 - 0.5 |
| Coating and finishing polymer | 10 - 20 |
| Purified water* | cbp |
| Total | |

By virtue of the foregoing, the present invention provides oral pharmaceutical compositions comprising ketorolac and B-complex in a single dose unit, without compromising their release, the compositions are useful in the treatment of moderate to severe pain, while reducing incidence and severity of adverse side effects.

The invention has been sufficiently disclosed so that a person with ordinary skill in the art can reproduce and obtain the results as recited in the present disclosure. Nonetheless, any person skilled in the art of the present invention may be able to make modifications thereto, which changes are not described specifically in the present invention. However, if application of said modifications in a given composition requires the subject matter as claimed in the following claims, said compositions should be considered as being within the scope of the present invention.

## Claims

1. A pharmaceutical composition for oral administration in compressed form as tablets, **characterized in that** it comprises:
a first compartment comprising therapeutically effective amounts of ketorolac, vitamin B1 and vitamin B12, or pharmaceutically acceptable salts thereof, compressibility vehicle, diluent binder, antistatic agent, lubricant, plasticizer and disintegrant;
a second compartment comprising either a coating or an isolating layer made from a coating polymer; and
a third compartment comprising pyridoxine or pharmaceutically acceptable salts thereof, and ligand binder polymer.

2. The pharmaceutical composition according to claim 1, further comprising a final coating made from a coating and finishing polymer.

3. The pharmaceutical composition according to claim 1, wherein said composition comprises from 2 to 120 mg of said ketorolac salt per unit dose; from 15 to 250 mg of said vitamin B1 salt per unit dose; from 15 to 250 mg of said vitamin B6 salt per unit dose; and from 0.1 to 10 mg of said vitamin B12 salt per unit dose.

4. The pharmaceutical composition according to claim 1, wherein said composition comprises from from 15.0 to 60.0% of said compressibility vehicle by weight of composition; from 5.0 to 60.0% of said diluent binder by weight of composition; from 0.002 to 1.0% of said antistatic agent by weight of composition; from 0.5 to 2.5% of said disintegrant by weight of composition; from 0.5 to 2.5% of said lubricant by weight of composition; from 0.2 to 11.5% of said plasticizer by weight of composition; from 5.0 to 40.0% of said ligand binder polymer by weight of composition; and from 1.5 to 7.5% of said coating polymer by weight of composition.

5. The pharmaceutical composition according to claim 2, wherein said composition comprises from 1.0 to 3.0 mg of said coating and finishing polymer per unit dose.

6. The pharmaceutical composition according to claim 1, wherein said composition comprises 5 mg of said ketorolac salt, 50 mg of said vitamin B1, 50 mg of said vitamin B6, and 1 mg of said vitamin B12 per dose unit.

7. A process for preparing the pharmaceutical composition according to claim 1, said process comprising the steps of:
a) mixing and compressing ketorolac, vitamin B1 and vitamin B12, or pharmaceutically acceptable salts thereof, compressibility vehicle, diluent binder, antistatic agent, lubricant, plasticizer and disintegrant;
b) coating of the compressed pellet obtained from a) with the coating polymer; and
c) coating said compressed pellet with a solution containing vitamin B6 or a pharmaceutically acceptable salt thereof, and ligand binder polymer.

8. Use of the pharmaceutical composition of claim 1 in the manufacture of a drug for the treatment of moderate to severe pain.

9. Use of the pharmaceutical composition of claim 1 in the manufacture of a drug for the treatment of neuralgia in different sites.

10. A pharmaceutical composition for oral administration of formulations as microcapsules comprising:
a first compartment comprising inert cores, therapeutically effective amounts of ketorolac, vitamin B1 and vitamin B12, or pharmaceutically acceptable salts thereof, ligand binder polymer, antioxidant sequestrant and antioxidant; and
a second compartment comprising vitamin B6 or pharmaceutically acceptable salts thereof, and coating polymer.

11. The pharmaceutical composition according to claim 10, further comprising a final coating made from a coating and finishing polymer.

12. The pharmaceutical composition according to claim 10, wherein said composition comprises from 2 to 120 mg of said ketorolac salt per unit dose; from 15 to 250 mg of said vitamin B1 salt per unit dose; from 15 to 250 mg of said vitamin B6 salt per unit dose; from 0.1 to 10 mg of said vitamin B12 salt per unit dose; from 160 to 400 mg of said inert cores per unit dose; from 10 to 20 mg of said coating polymer per unit dose; from 0.02 to 0.5 mg of said antioxidant sequestrant per unit dose; from 0.02 to 0.5 mg of said ligand binder polymer per unit dose; from 0.001 to 0.5 mg of said antioxidant per unit dose.

13. The pharmaceutical composition according to claim 11, wherein said composition comprises from 10 to 20 mg of said coating and finishing polymer per unit dose.

14. Use of the pharmaceutical composition of claim 10 in the manufacture of a drug for the treatment of moderate to severe pain.

15. Use of the pharmaceutical composition of claim 10 in the manufacture of a drug for the treatment of neuralgia in different locations.

## Patentansprüche

1. Pharmazeutische Zusammensetzung zur oralen Verabreichung in komprimierter Form als Tabletten,
**dadurch gekennzeichnet,**
**dass** die Zusammensetzung Folgendes umfasst:
einen ersten Abschnitt, der therapeutisch wirksame Mengen von Ketorolac, Vitamin B1 und Vitamin B12 oder pharmazeutisch verträgliche Salze derselben, Kompressibilitätsmittel, Lösungsbindemittel, Antistatikum, Gleitmittel, Weichmacher und Abbaumittel umfasst,
einen zweiten Abschnitt, der entweder eine Beschichtung oder eine Isolierschicht aus einem Beschichtungspolymer umfasst, und
einen dritten Abschnitt, der Pyridoxin oder pharmazeutisch verträgliche Salze desselben und ein Liganden-Bindemittelpolymer (engl.: *ligand binder polymer*) umfasst.

2. Pharmazeutische Zusammensetzung nach Anspruch 1, des Weiteren umfassend eine Endbeschichtung aus einem Beschichtungs- und Finishing-Polymer.

3. Pharmazeutische Zusammensetzung nach Anspruch 1, wobei die Zusammensetzung 2 bis 120 mg des Ketorolacsalzes pro Einzeldosis, 15 bis 250 mg des Vitamin-B1-Salzes pro Einzeldosis, 15 bis 250 mg des Vitamin-B6-Salzes pro Einzeldosis und 0,1 bis 10 mg des Vitamin-B12-Salzes pro Einzeldosis umfasst.

4. Pharmazeutische Zusammensetzung nach Anspruch 1, wobei die Zusammensetzung bezogen auf das Gewicht der Zusammensetzung 15,0 bis 60,0% Kompressibilitätsmittel, bezogen auf das Gewicht der Zusammensetzung 5,0 bis 60,0% Lösungsbindemittel, bezogen auf das Gewicht der Zusammensetzung 0,002 bis 1,0% Antistatikum, bezogen auf das Gewicht der Zusammensetzung 0,5 bis 2,5% Abbaumittel, bezogen auf das Gewicht der Zusammensetzung 0,5 bis 2,5% Gleitmittel, bezogen auf das Gewicht der Zusammensetzung 0,2 bis 11,5% Weichmacher, bezogen auf das Gewicht der Zusammensetzung 5,0 bis 40,0% Liganden-Bindemittelpolymer und bezogen auf das Gewicht der Zusammensetzung 1,5 bis 7,5% Beschichtungspolymer umfasst.

5. Pharmazeutische Zusammensetzung nach Anspruch 2, wobei die Zusammensetzung 1,0 bis 3,0 mg Beschichtungs- und Finishing-Polymer pro Einzeldosis umfasst.

6. Pharmazeutische Zusammensetzung nach Anspruch 1, wobei die Zusammensetzung 5 mg Ketorolacsalz, 50 mg Vitamin B1, 50 mg Vitamin B6 und 1 mg Vitamin B12 pro Einzeldosis umfasst.

7. Verfahren zum Herstellen der pharmazeutischen Zusammensetzung nach Anspruch 1, wobei das Verfahren die folgenden Schritte umfasst:
a) Mischen und Pressen von Keterolac, Vitamin B1 und Vitamin B12 oder von pharmazeutisch verträglichen Salzen derselben, Kompressibilitätsmittel, Lösungsbindemittel, Antistatikum, Gleitmittel, Weichmacher und Abbaumittel;
b) Beschichten des aus a) erhaltenen gepressten Pellets mit dem Beschichtungspolymer; und
c) Beschichten des gepressten Pellets mit einer Lösung, die Vitamin B6 oder ein pharmazeutisch verträgliches Salz desselben enthält, und einem Liganden-Bindemittelpolymer.

8. Verwendung der pharmazeutischen Zusammensetzung nach Anspruch 1 in der Herstellung eines Medikaments zur Behandlung von mäßigen bis starken Schmerzen.

9. Verwendung der pharmazeutischen Zusammensetzung nach Anspruch 1 in der Herstellung eines Medikaments zur Behandlung von Neuralgien an verschiedenen Orten.

10. Pharmazeutische Zusammensetzung zur oralen Verabreichung von Formulierungen als Mikrokapseln, umfassend:
einen ersten Abschnitt, der inerte Kerne, therapeutisch wirksame Mengen von Keterolac, Vitamin B1 und Vitamin B12 oder
pharmazeutisch verträgliche Salze derselben, Liganden-Bindemittelpolymer, Antioxidanskomplexbildner (engl.: *antioxidant sequestrant*) und Antioxidans umfasst, und
einen zweiten Abschnitt, der Vitamin B6 oder pharmazeutisch verträgliche Salze desselben und Beschichtungspolymer enthält.

11. Pharmazeutische Zusammensetzung nach Anspruch 10, des Weiteren umfassend eine Endbeschichtung aus einem Beschichtungs- und Finishing-Polymer.

12. Pharmazeutische Zusammensetzung nach Anspruch 10, wobei die Zusammensetzung 2 bis 120 mg des Ketorolacsalzes pro Einzeldosis, 15 bis 250 mg des Vitamin-B1-Salzes pro Einzeldosis, 15 bis 250 mg des Vitamin-B6-Salzes pro Einzeldosis, 0,1 bis 10 mg des Vitamin-B12-Salzes pro Einzeldosis, 160 bis 400 mg der inerten Kerne pro Einzeldosis, 10 bis 20 mg des Beschichtungspolymers pro Einzeldosis, 0,02 bis 0,5 mg des Antioxidanskomplexbildners pro Einzeldosis, 0,02 bis 0,5 mg des Liganden-Bindemittelpolymers pro Einzeldosis und 0,001 bis 0,5 mg des Antioxidans pro Einzeldosis umfasst.

13. Pharmazeutische Zusammensetzung nach Anspruch 11, wobei die Zusammensetzung 10 bis 20 mg des Beschichtungs- und Finishing-Polymers pro Einzeldosis umfasst.

14. Verwendung der pharmazeutischen Zusammensetzung nach Anspruch 10 in der Herstellung eines Medikaments zur Behandlung von mäßigen bis starken Schmerzen.

15. Verwendung der pharmazeutischen Zusammensetzung nach Anspruch 10 in der Herstellung eines Medikaments zur Behandlung von Neuralgien an verschiedenen Orten.

## Revendications

1. Une composition pharmaceutique pour administration orale sous forme compressée en comprimés, **caractérisée en ce qu'**elle comprend:
- un premier compartiment comprenant des quantités thérapeutiquement efficaces de kétorolac, de vitamine B1 et de vitamine B12, ou des sels pharmaceutiquement acceptables en dérivant, un excipient de compressibilité, un liant diluant, un agent antistatique, un lubrifiant, un plastifiant et un délitant;
- un second compartiment comprenant soit un revêtement soit une couche isolante formée à partir d'un polymère d'enrobage, et
- un troisième compartiment comprenant de la pyridoxine ou des sels pharmaceutiquement acceptables en dérivant, et un polymère liant ligand.

2. La composition pharmaceutique selon la revendication 1, comprenant en outre un revêtement final formé à partir d'un revêtement et d'un polymère de finition.

3. La composition pharmaceutique selon la revendication 1, dans laquelle ladite composition comprend :
- de 2 à 120 mg dudit sel de kétorolac par dose unitaire,
- de 15 à 250 mg dudit sel de vitamine B1 par dose unitaire,
- de 15 à 250 mg dudit sel de vitamine B6 par dose unitaire et
- de 0,1 à 10 mg dudit sel de vitamine B12 par dose unitaire.

4. La composition pharmaceutique selon la revendication 1, dans laquelle ladite composition comprend :
- de 15,0 à 60,0% dudit excipient de compressibilité, en poids de la composition,
- de 5,0 à 60,0% dudit liant diluant, en poids de la composition,
- de 0,002 à 1,0% dudit agent antistatique, en poids de la composition,
- de 0,5 à 2,5% dudit délitant, en poids de la composition,
- de 0,5 à 2,5% dudit lubrifiant, en poids de la composition,
- de 0,2 à 11,5% dudit plastifiant, en poids de la composition,
- de 5,0 à 40,0% dudit polymère liant ligand, en poids de la composition, et
- de 1,5 à 7,5% dudit polymère d'enrobage, par poids de la composition.

5. La composition pharmaceutique selon la revendication 2, dans laquelle ladite composition comprend de 1,0 à 3,0 mg dudit revêtement et polymère de finition par dose unitaire.

6. La composition pharmaceutique selon la revendication 1, dans laquelle ladite composition comprend 5 mg dudit sel de kétorolac, 50 mg de ladite vitamine B1, 50 mg de ladite vitamine B6, et 1 mg de ladite vitamine B12 par dose unitaire.

7. Un procédé de préparation de la composition pharmaceutique selon la revendication 1, ledit procédé comprenant les étapes:
a) mélanger et comprimer le kétorolac, la vitamine B1 et la vitamine B12, ou les sels pharmaceutiquement acceptables en dérivant, l'excipient de compressibilité, le liant diluant, l'agent antistatique, le lubrifiant, le plastifiant et le délitant;
b) revêtir la pastille compressée obtenue à partir de a) avec le polymère d'enrobage,
et
c) revêtir ladite pastille compressée avec une solution contenant de la vitamine B6 ou un sel pharmaceutiquement acceptable en dérivant, et un polymère liant ligand.

8. Utilisation de la composition pharmaceutique selon la revendication 1, dans la fabrication d'un médicament pour le traitement de la douleur modérée à sévère.

9. Utilisation de la composition pharmaceutique selon la revendication 1, dans la fabrication d'un médicament pour le traitement des névralgies sur des zones différentes.

10. Une composition pharmaceutique pour administration orale de formulations sous forme de microcapsules comprenant:
- un premier compartiment comprenant des noyaux inertes, des quantités thérapeutiquement efficaces de kétorolac, de vitamine B1 et de vitamine B12, ou des sels pharmaceutiquement acceptables en dérivant, un polymère liant ligand, un séquestrant antioxydant et un antioxydant, et
- un second compartiment comprenant de la vitamine B6 ou des sels pharmaceutiquement acceptables en dérivant, et un polymère d'enrobage.

11. La composition pharmaceutique selon la revendication 10, comprenant en outre un revêtement final formé à partir d'un revêtement et d'un polymère de finition.

12. La composition pharmaceutique selon la revendication 10, dans laquelle ladite composition comprend :
de 2 à 120 mg dudit sel de kétorolac par dose unitaire,
de 15 à 250 mg dudit sel de vitamine B1 par dose unitaire,
de 15 à 250 mg dudit sel de vitamine B6 par dose unitaire,
de 0,1 à 10 mg dudit sel de vitamine B12 par dose unitaire,
de 160 à 400 mg desdits noyaux inertes par dose unitaire,
de 10 à 20 mg dudit polymère d'enrobage par dose unitaire,
de 0,02 à 0,5 mg dudit séquestrant antioxydant par dose unitaire,
de 0,02 à 0,5 mg dudit polymère liant ligand par dose unitaire, et
de 0,001 à 0,5 mg dudit antioxydant par dose unitaire.

13. La composition pharmaceutique selon la revendication 11, dans laquelle ladite composition comprend de 10 à 20 mg dudit revêtement et polymère de finition par dose unitaire.

14. Utilisation de la composition pharmaceutique selon la revendication 10 dans la fabrication d'un médicament pour le traitement de la douleur modérée à sévère.

15. Utilisation de la composition pharmaceutique selon la revendication 10 dans la fabrication d'un médicament pour le traitement des névralgies sur des zones différentes.
